# EUROPEAN PATENT SPECIFICATION

(11) **EP 0 497 987 B1**
(45) Date of publication and mention of the grant of the patent: **25.03.1998**
(21) Application number: 91914677.9
(22) Date of filing: 27.08.1991
(51) Int. Cl.: C08G 59/62, C08G 59/40, C08L 63/00, C09D 163/00, C09J 163/00, H01L 23/29, B32B 27/38

(54) **EPOXY RESIN COMPOSITION**
EPOXYDHARZZUSAMMENSETZUNG
COMPOSITION DE RESINE EPOXY

(30) Priority: 27.08.1990 JP 222407/90; 10.07.1991 JP 170161/91
(43) Date of publication of application: 12.08.1992
(73) Proprietor: FUJITSU LIMITED, Kawasaki-shi, Kanagawa 211 (JP)
(72) Inventor: TAKIGAWA, Yukio, Nakahara-ku, Kawasaki-shi, Kanagawa 211 (JP); SAWATARI, Norio, Nakahara-ku, Kawasaki-shi, Kanagawa 211 (JP); NAKATA, Yoshihiro, Nakahara-ku, Kawasaki-shi, Kanagawa 211 (JP)
(74) Representative: Silverman, Warren
(86) International application number: JP9101134
(87) International publication number: WO9203489

(56) References cited:
- EP-A- 0 361 438
- GB-A- 788 397
- JP-A- 5 341 399
- JP-A-54 155 297
- JP-A-55 145 730
- JP-A-63 291 919

## Description

This invention relates to an epoxy resin composition. More particularly, this invention relates to an epoxy resin composition containing an epoxy resin as a substratal resin (main component). Further, this invention relates to an epoxy resin composition having a particularly excellent heat resistance, toughness, flexibility, resistance to cracking, mold releasability, and hydrophobicity. Since the epoxy resin composition of this invention possesses the outstanding properties as described above, it can be utilized advantageously in various fields, particularly in the fields of multilayer laminate resins, electroconductive pastes, protective films for electronic devices, adhesive agents, coating materials, sealing materials, and molding materials.

Recent electronic and electric devices and transportation devices are becoming smaller and lighter in weight and have an increased performance, and this trend has emphasized the need for a material having an excellent heatproofing.

Polyimide resins are universally renowned as a heatproof resin, but since this resin is the product of a dehydration-condensation, the produced cured resin is liable to contain voids due to the water of condensation that occurs as a consequence of the reaction, and these voids impair the reliability of the cured resin product. The polyimide itself is insoluble and infusible, and therefore, is not easily moldable.

As polyimides having an improved moldability, maleimide resins such as bismaleimide and polymaleimide resins are known in the art. Bismaleimide, however, has a high melting point and therefore, has the disadvantage of a poor workability because the curing during the process of molding requires protracted application of a high temperature generally exceeding 200°C (180 to 350°C and 15 to 16 minutes). Bismaleimide is further disadvantageous in that it has a poor hydrophobicity and consequently, has a conspicuously poor reliability as the cured resin product. Polymaleimide has a curing temperature about 70 to 80°C lower than that of bismaleimide, but the cured product of the polymaleimide has a disadvantage in that it is easily cracked and embrittled because of a high cross-link density and large residual strain. It is, indeed, known from US-A-4,131,632 to improve the heat resistance of maleimide resins by including therein an allyl bisphenol compound of the indicated general formula, and an epoxy resin.

The practice of using an epoxy resin having an excellent moldability, in the place completely of the maleimide resin, is also known in the art. Where the epoxy resin is used as a substratal resin, an amine, an acid anhydride, or a phenol is generally used in combination with the epoxy resin as a curing agent therefor, but the cured product obtained has an unsatisfactory resistance to heat. The epoxy/phenol cured system, for example, typically has a glass transition point generally of from 140 to 170°C, and this glass transition point should be raised to a level above 190°C, preferably above 200°C. This particular requirement may be met according to EP-A-0 361 438 by inclusion of a polyallyl phenol, but there are a number of further requirements which should be met by epoxy resins for use, *inter alia,* in production of substrates for printed circuit boards.

A major object of this invention is thus to overcome the drawbacks of the prior art described above, and for this purpose, to provide an epoxy resin composition having an excellent resistance to heat, toughness, flexibility, cracking resistance, mold releasability, and hydrophobicity, and being consequently, advantageously usable in various fields.

The object described above is accomplished by an epoxy resin composition containing an epoxy resin and a polyallyl phenol curing agent possessing in the repeating unit thereof a plurality of component units represented by the following formula I: 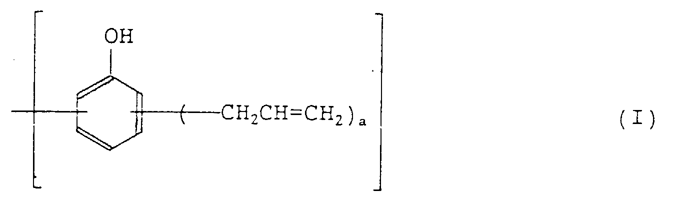 wherein a is an integer of 1 to 4 inclusive,
characterized in that said epoxy resin is a biphenyl epoxy resin, said polyallyl phenol curing agent is contained in an amount of 30 to 120 parts by weight based on 100 parts by weight of said biphenyl epoxy resin, said epoxy resin composition further contains as a flexibility-imparting agent a silicon-modified epoxy resin in an amount of 5 to 80 parts by weight based on 100 parts by weight of said biphenyl epoxy resin and an additional component selected from butadiene/acrylonitrile copolymer, polystyrene /polybutadiene/polystyrene terminal block copolymer, ethylene/propylene type terpolymers, ethylene/α-olefin copolymers, and epoxy group-containing silicone rubber powder is either absent or is present in an amount of from 5 to 80% by weight, based on the weight of the said biphenyl epoxy resin.

In the description which follows, reference will be made to the accompanying drawings, wherein:-
Figs. 1 to 8 are graphs showing the relationship between the time of PCT (Pressure Cooker Test) treatment (h) and the fraction which is defective (%) obtained in a relevant working example,
Fig. 9 is a schematic diagram of an aluminiumwired model device used for evaluation of moistureproofing;
Fig. 10 compares products of the prior art and the products of this invention with respect to the relationship between the time of PCT treatment (h) and the fraction which is defective (%);
Fig. 11 is a graph showing the effect of the number of allyl groups on the surface of close contact.

The biphenyl epoxy resin to be used as the substratal resin in epoxy compositions of this invention is not particularly limited. According to the knowledge acquired by the inventors, the epoxy resins advantageously usable herein include tetramethyl biphenyl type epoxy resins which have at least two epoxy groups in the molecular unit thereof. These epoxy resins may be used either independently or in the form of a mixture of two or more members.

The biphenyl type epoxy resin used in these experiments will generally be of the type disclosed in Japanese Unexamined Patent Publication No. 251,419/1988. The resin is represented by the following general formula: 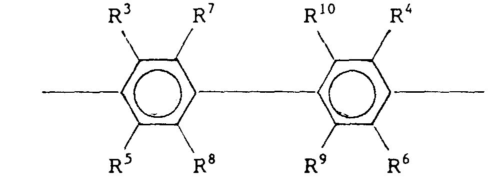 (wherein R³ to R¹⁰ each stand for a hydrogen, a lower alkyl group of one to four carbon atoms, or a halogen atom). Desirable examples of R³ to R¹⁰ are hydrogen atom, methyl group, ethyl group, propyl group, butyl group, chlorine atom, and bromine atom.

Desirable concrete examples of such epoxy resins disclosed in Japanese Unexamined Patent Publication No. 251,419/1988 are the polymers of 4,4'-bis(2,3-epoxypropoxy)biphenyl, 4,4'-bis(2,3-epoxypropoxy)-3,3',5,5'-tetramethyl biphenyl, 4,4'-bis(2,3-epoxypropoxy)-3,3',5,5'-tetramethyl-2-chlorobiphenyl, 4,4'-bis(2,3-epoxypropoxy)-3,3',5,5'-tetramethyl-2-bromobiphenyl, 4,4'-bis(2,3-epoxypropoxy)-3,3',5,5'-tetraethylbiphenyl, and 4,4'-bis(2,3-epoxypropoxy)-3,3',5,5'-tetrabutylbiphenyl. These epoxy resins are available commercially. The product of Yuka-Shell Epoxy K.K. marketed under the product name "YX-4000H" is one example.

Polyallyl phenols advantageously usable as the curing agent in the epoxy resin composition of this invention are represented by the following formulas II, III, IV, and V: 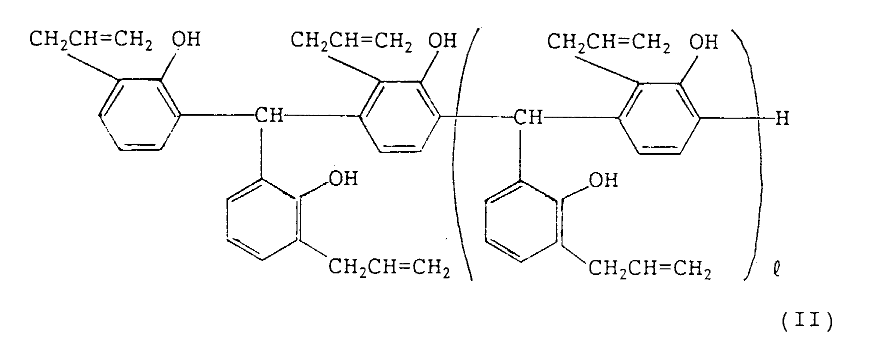 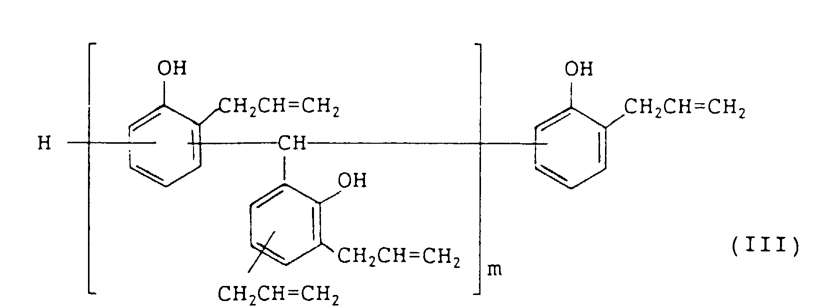 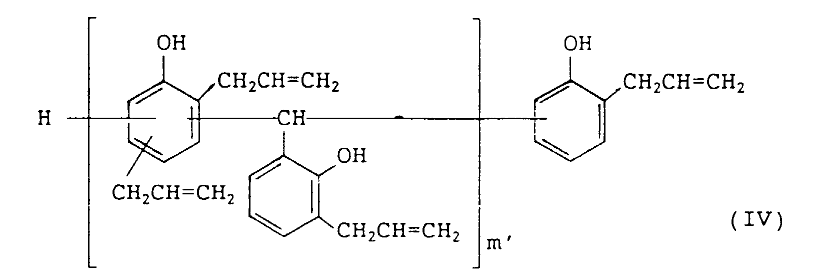 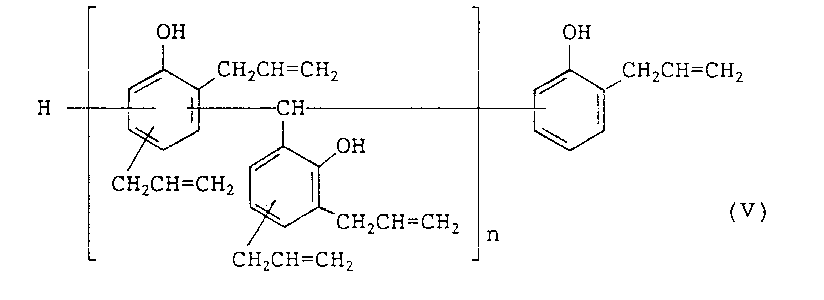 (wherein ℓ is 0 or an integer of 1 to 4 inclusive, m and m' each are an integer of 1 to 4 inclusive, and n is an integer of 1 to 4 inclusive). Here, it is understood that these polyallyl phenols have a better viscosity, workability, and improved heatproofing in proportion to an increase in the numerical values of l, m, and n in the formulas. These polyallyl phenols may be synthesized by a known method, or may be produced commercially. The product of Mitsubishi Petro-Chemical Co., Ltd. marketed under product name of "SH-150AR" is one example.

By the use of a polyallyl phenol as the curing agent, the epoxy resin composition enables the cured resin product thereof to acquire an elevated glass transition point of from 180 to 220°C, an improved heatproofing, and an augmented hydrophobicity. The merits are ascribable to the effects of the allyl group possessed by the polyallyl phenol. The amount of the polyallyl phenol to be incorporated in the epoxy resin composition is preferably from about 30 to about 120 parts by weight, based on 100 parts by weight of the epoxy resin. If the amount of the polyallyl phenol to be incorporated is less than 30 parts by weight, the curing reaction of the epoxy resin composition does not proceed sufficiently. If this amount exceeds 120 parts by weight, the cured resin product has a poor heatproofing.

In this invention, for the purpose of conferring flexibility on the cured resin product of the epoxy resin composition, it is essential for the epoxy resin composition to be used in combination with a flexibility-imparting agent which is a silicon-modified epoxy resin optionally used together with a butadiene/acrylonitrile copolymer, polystyrene/polybutadiene/polystyrene terminal block copolymer, ethylene/propylene type terpolymer, ethylene/α-olefin copolymer, or an epoxy functional group-containing silicone rubber powder.

It has been found that the addition of a silicon-modified epoxy resin to the substratal resin is effective in improving the cured resin product simultaneously in the properties of a resistance to cracking, flexibility, and mold releasability, and in improving the epoxy resin composition itself from the viewpoint of heatproofing and waterproofing, compared with the corresponding unmodified epoxy resin composition. Silicon-modified epoxy resins of varying forms are usable for this purpose. A resin available from Dai-Nippon Ink & Chemicals, Inc. marketed under product code of "SIN-620" can be advantageously used, for example. This silicon-modified epoxy resin is to be used in the epoxy resin composition in an amount which may range from 5 to 80 parts by weight, based on 100 parts by weight of the epoxy resin depending on the effect desired from the addition thereof. The reason for this particular range, as specifically demonstrated in the working examples to be cited hereinbelow, is that the effect of the addition is not manifested if this amount is less than 5 parts by weight and the heatproofing of the resin composition is impaired by a layer separation of the amount exceeds 80 parts by weight.

Imparting of a satisfactory flexibility to the cured resin product can be attained by the addition of the silicon-modified epoxy resin. When the flexibility so imparted by the added silicon-modified epoxy resin is not sufficient, it can be improved by the additional use of a butadiene/acrylonitrile copolymer. The butadiene/acrylonitrile copolymer useful for this purpose is represented by the following formula. 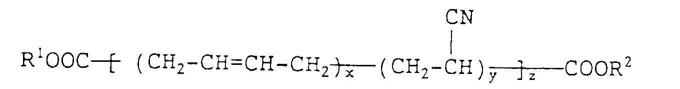 (wherein R¹ and R² each stand for a hydrogen atom or a hydrocarbon group such as alkyl group or aryl group, x and y stand for numerical values satisfying the expression, 1 ≦ x/y ≦ 20, and z stands for an integer of from 5 to 20 inclusive).

A polystyrene/polybutadiene/polystyrene terminal block copolymer, an ethylene/propylene type terpolymer, an ethylene/α-olefin copolymer, or an epoxy functional group-containing silicone rubber may also be added, when necessary for an improvement of the flexibility.

These flexibility-imparting agents may possess various structures, and are marketed in various forms. The product of Shell Chemical K.K. marketed under the trademark of "Kraton® G-1652" is available as a polystyrene/polybutadiene/polystyrene terminal block copolymer, the product of Japan Synthetic Rubber Co., Ltd. marketed under a product name of "JSR57P" as an ethylene/propylene type terpolymer, the product of Mitsui Petrochemical Industries, Ltd. marketed under the trademark of "Tafmer®-P P-0280" as an ethylene/α-olefin copolymer, and the product of Toray-Dow Corning K.K. marketed under the trademark of "Torayfil® E-601" as an epoxy-functional group-containing silicon-rubber, for example. These copolymers are effective in improving the toughness and flexibility of the cured resin product, and thus the resistance to cracking.

In the epoxy resin composition, the copolymer which can be added additionally to the silicon-modified epoxy resin can be used in a varying amount depending on the effect desired to be derived from its addition. Although this amount is variable in a wide range, it is generally desired to be approximately from 5 to 80 parts by weight, based on 100 parts by weight of the epoxy resin. The reason for this particular range, as demonstrated in the working examples to be cited hereinbelow, is that the effect of the addition is not manifested if the amount is less than 5 parts by weight and the cured resin product has a poor heat-proofing if the amount exceeds 80 parts by weight.

The epoxy resin composition of this invention may contain therein a powdery inorganic component (such as, for example, fused silica, crystalline silica, alumina, and calcium carbonate) as a filler in an amount of from 30 to 85% by weight, based on the total amount of the composition. This inorganic filler fails to manifest the effect of its addition if this amount is less than 30% by weight. Conversely, if this amount exceeds 85% by weight, the epoxy resin composition has a poor flowability, and consequently, workability.

Further, the epoxy resin composition according to this invention may incorporate therein the following components when necessary.
(1) A catalyst for promoting the curing reaction of epoxy resin with polyallyl phenol. The agents usable effectively herein include imidazole type compounds represented by 2-methyl imidazole, phosphine type compounds represented by triphenyl phosphine, and DBU(diazabicycloundecene) type compounds represented by phenol salts of DBU, for example.
(2) A coupling agent for enhancing the compatibility of the inorganic filler with the resin during the addition of the filler to the resin. The fillers effectively usable herein include silane type coupling agents represented by 3-aminopropyltriethoxysilane and titanium type coupling agents represented by tetraoctyl bis(phosphite) titanate, for example.
   Although the amount of the coupling agent is variable with the kind, amount, and specific surface area of the inorganic filler to be used and the minimum covering area of the coupling agent, it is desired to be approximately from 0.1 to 15 parts by weight in this invention.
(3) A mold release auxiliary agent selected from carnauba wax, stearic acid and metal salts thereof, montan wax, etc., a flame-retarding agent selected from among epoxy bromide resin, antimony trioxide, etc., and a colouring agent or pigment such as carbon or titanium dioxide may be added.

The epoxy resin composition of this invention can be prepared by having the components mentioned above kneaded at a temperature of approximately from 60 to 120°C by the use of a roll kneader or an extruder, for example. Further, in this invention, the shaped article obtained by molding and curing the epoxy resin composition is desirably given an aftercuring treatment for completing the curing reaction of the epoxy resin still proceeding in the cured resin product. The aftercuring treatment subsequent to the step of molding can be carried out by heat-treating the cured resin product for a prescribed time in a constant temperature bath kept at a temperature substantially equaling the temperature used in the molding treatment.

In the epoxy resin composition of this invention, the polyallyl phenol used as a curing agent acts on the epoxy resin used as a substratal resin effectively improving the heatproofing and hydrophobicity of the cured resin product, and the specific silicon-modified resin, optionally with one member selected from among silicon-modified epoxy resin, butadiene-acrylonitrile copolymer, polystyrene/polybutadiene/polystyrene terminal block copolymer, ethylene/propylene type rubber, ethylene/α-olefin copolymers, and epoxy functional group-containing silicone rubber powder and acts as a flexibility-imparting agent and effectively functions as an agent for enhancing the cracking resistance and mold releasability. Thus, these additives simultaneously improve the flame-retarding property, flexibility, mold releasability, and cracking resistance. Further, the addition of the inorganic filler enhances the mechanical strength.

Now, this invention will be described more specifically with reference to working examples and comparative examples. Wherever "parts" is mentioned in the following examples, it will be construed as referring to "parts by weight" unless otherwise specified.

### Examples 1 to 9 and Comparative Examples 1 to 4:

The experiments covered herein represent cases using an epoxy resin composition comprising an epoxy resin, a polyallyl phenol, and a silicon-modified epoxy resin.

The following raw materials were used in these experiments.
- Epoxy resin:: A biphenyl type epoxy resin (produced by Yuka-Shell Epoxy K.K. and marketed under product code of "YX-4000H)
- Polyallyl phenol:: A product of Mitsubishi Petro-Chemical Co., Ltd. marketed under product code of "SH-150AR"
- Silicon-modified epoxy resin:: A product of Dai-Nippon Ink & Chemicals, Inc. marketed under product code of "SIN-620"

A pertinent composition was prepared by placing these raw materials in a varying ratio indicated in Table 1 in a pressure kneader and kneading them simultaneously therein. A test piece of the composition was produced as follows.

First, the composition resulting from the kneading was comminuted into a 8-mesh pass powder. This powder was placed in a press mold and compression molded therein at 200°C under 80 kg/cm² for 20 minutes. The shaped mass thus obtained was subjected to an after-curing treatment at 200°C for eight hours.

The composition produced as described above was tested for various properties as follows.
- Glass transition point:: This property was determined with a thermomechanical analyzer (produced by Shinku Riko K.K.)
- Flexural strength:: This property was determined in accordance with the method specified in Japanese Industrial Standard (JIS) K-6911.
- Crack:: The occurrence of this phenomenon in a given test piece and the extent of the phenomenon were determined by observing a cross section of a given test piece (10 × 5 × 30 mm) under a microscope.
- Absorbing ratio:: This property was determined by the method (absorption of boiling water) specified in JIS K-6911.
- Mold releasability:: This property was determined by repeating the molding of a given composition on a chromium-plated plate, taking into count the cycles of the molding, and measuring the adhesive force.
- Moistureproofness:: This property was determined by subjecting a given sample to a PCT (pressure cooker test) treatment at 125°C under 2.3 atmospheres by the use of an aluminum-wired model device illustrated in Fig. 9 and testing the wire for electric resistance.

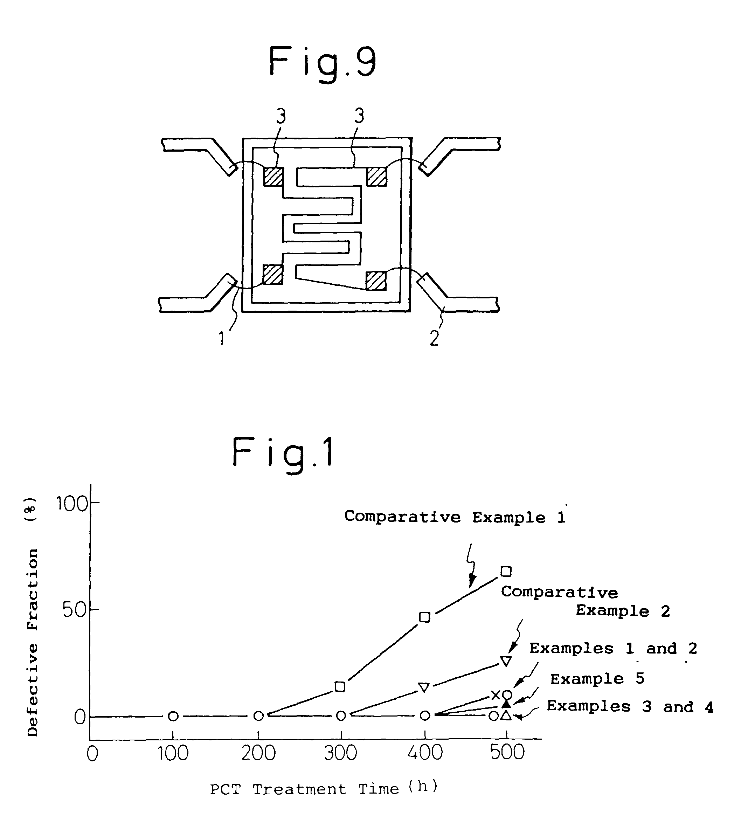

In Fig. 9, 1 stands for a gold wire, 2 for a lead, and 3 for an aluminum electrode. In the test, the circuit width and the circuit interval were respectively 10 µm and 10 µm.

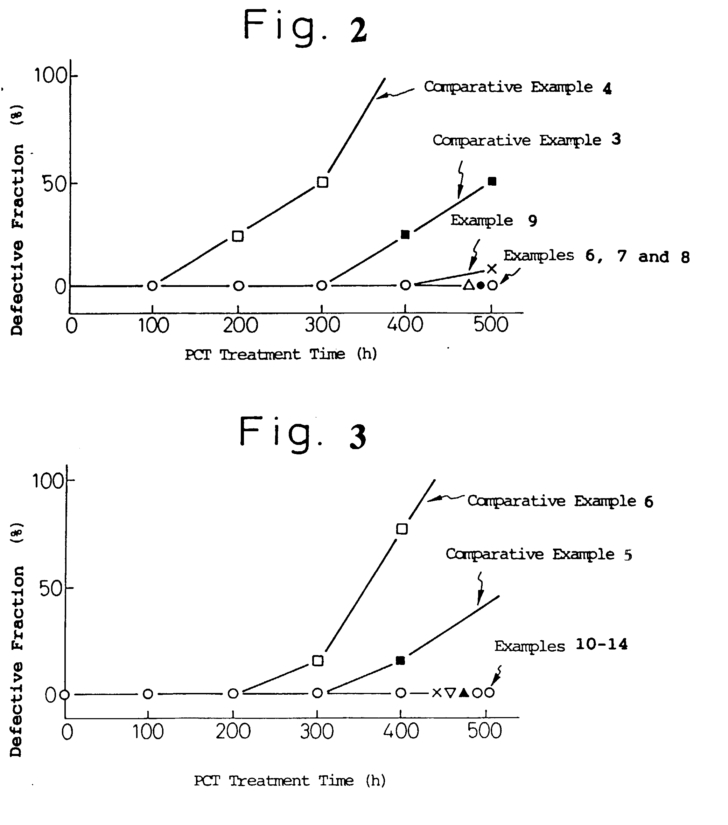

The results are shown in Table 1 and Fig. 1 and Fig. 2. It is clearly noted from these results, particularly the results of Examples 1 to 5 and Comparative Examples 1 and 2 that the addition of a silicon-modified epoxy resin improved the toughness, mold releasability, and flexibility of a cured resin product, that the desirable amounts of addition were from 5 to 80 parts by weight, and that the use of a polyallyl phenol as a curing agent allowed the production of a resin composition having an excellent hydrophobicity.

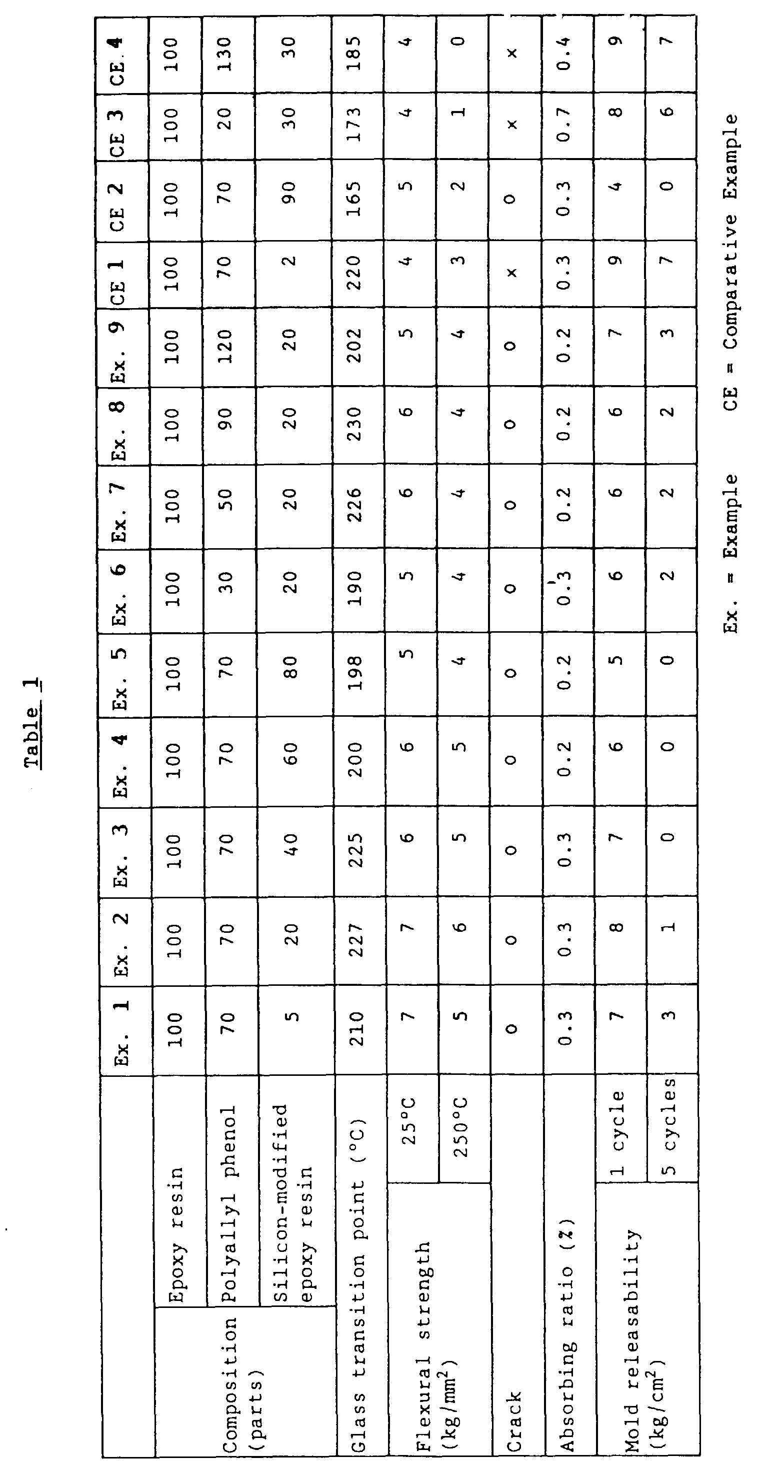

### Examples 10 to 14 and Comparative Examples 5 and 6:

The experiments covered herein represent cases using an epoxy resin composition comprising an epoxy resin, a polyallyl phenol, a silicon-modified epoxy resin, and a butadiene/acrylonitrile copolymer.

The following raw materials were used in these experiments.
- Epoxy resin:: A biphenyl type epoxy resin (produced by Yuka-Shell Epoxy K.K. and marketed under product code of "YX-4000H)
- Polyallyl phenol:: A product of Mitsubishi Petro-Chemical Co., Ltd. marketed under product code of "SH-150AR"
- Silicone-modified epoxy resin:: A product of Dai-Nippon Ink & Chemicals, Inc. marketed under product code of "SIN-620"
- Butadiene/acrylonitrile copolymer:: A product of Ube Industries, Ltd. marketed under designation "Hycar® CTBN1300"

A pertinent composition was prepared by placing the raw materials in a varying ratio indicated in Table 2 in a pressure kneader and kneading them simultaneously therein. Test pieces of these compositions and the tests for various properties were carried out in the same manner as in Examples 1 to 9. The compositions were also tested for bleedout and package crack as follows.
- Bleedout:: The occurrence of this phenomenon on a given test piece (10 × 5 × 30 mm) and the extent of the phenomenon were determined by visually observing the surface of the test piece.
- Package crack:: The test for this phenomenon was performed by allowing a produced package to absorb moisture under the conditions of 85°C and 85% RH for 96 hours, immersing this package in a solder bath at 260°C for 20 seconds, and then visually examining the package.

The results are shown in Table 2 and in Fig. 3. It is clearly noted from these results that the addition of the butadiene/acrylonitrile copolymer in an amount of from 5 to 80 parts by weight improved the flexibility and toughness of a cured resin product, and that the use of the polyallyl phenol as a curing agent allowed production of a resin composition having an excellent hydrophobicity.

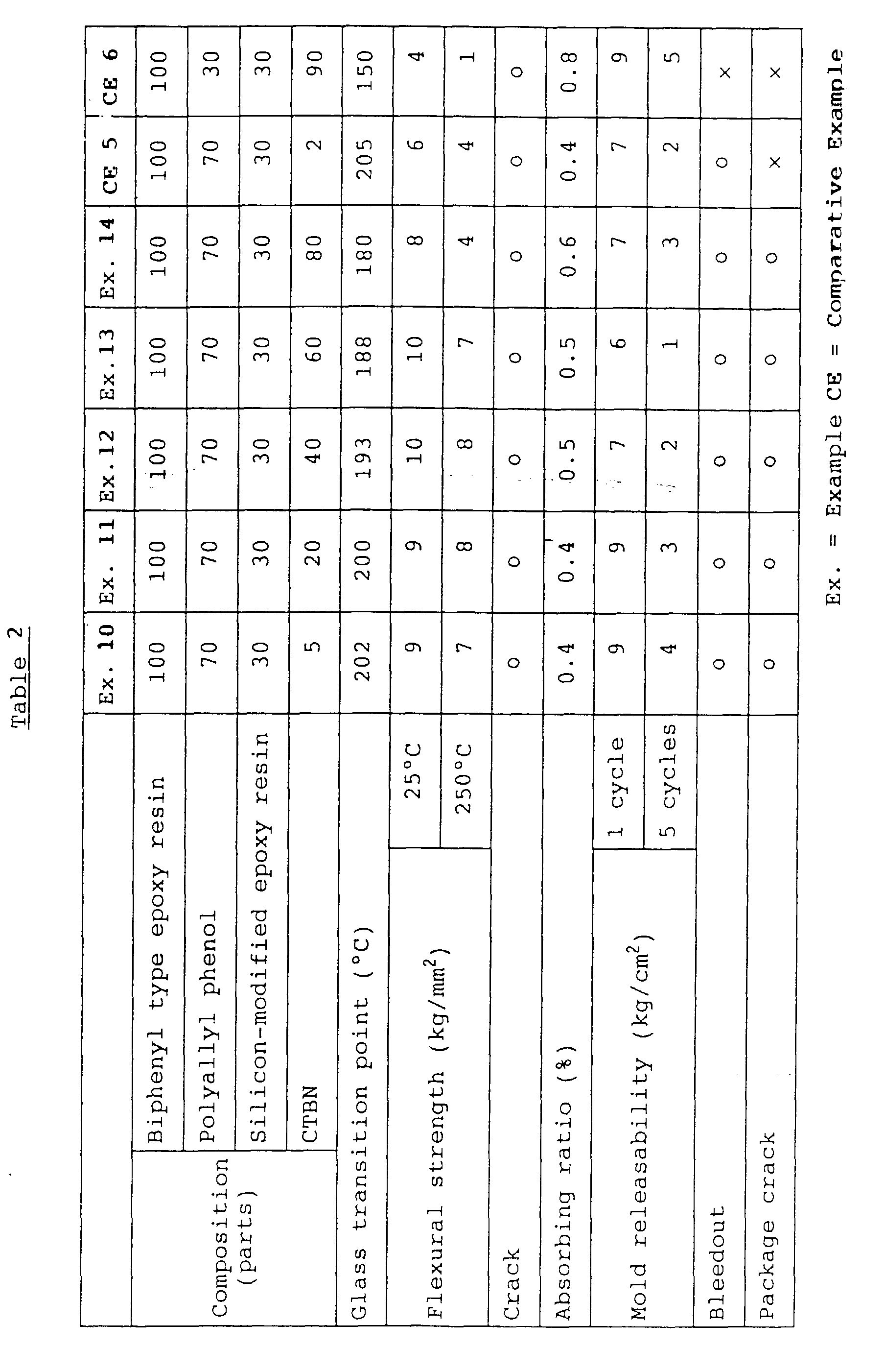

### Examples 15 to 19 and Comparative Examples 7 and 8:

The experiments covered herein represent cases using an epoxy resin composition comprising an epoxy resin, a polyallyl phenol, a silicon-modified epoxy resin, and a polystyrene/polybutadiene/polystyrene terminal block copolymer.

The following raw materials were used in these experiments.
- Epoxy resin:: A biphenyl type epoxy resin (produced by Yuka-Shell Epoxy K.K. marketed under product code of "YX-4000H")
- Polyallyl phenol:: A product of Mitsubishi Petro-Chemical Co., Ltd. marketed under product code "SH-150AR"
- Silicon-modified epoxy resin:: A product of Dai-Nippon Ink & Chemicals, Inc. marketed under product code "SIN-620"
- Polystyrene/polybutadiene/polystyrene terminal block copolymer:: A product of Shell Chemical K.K. marketed under the trademark of "Kraton G-1652"

A pertinent composition was prepared by placing these raw materials in a varying ratio indicated in Table 3 in a pressure kneader and kneading them simultaneously therein. Test pieces of these compositions and the tests for various properties were carried out in the same manner as in Examples 10 to 14. The test for package crack was varied, however, as follows.
- Package crack:: The test for this phenomenon was performed by molding an 84 pin GFP (quad flat package) allowing the package to absorb moisture at 121°C for 24 hours, immersing the package in a solder bath at 260°C for 20 seconds, and examining the package to detect cracks, if any.

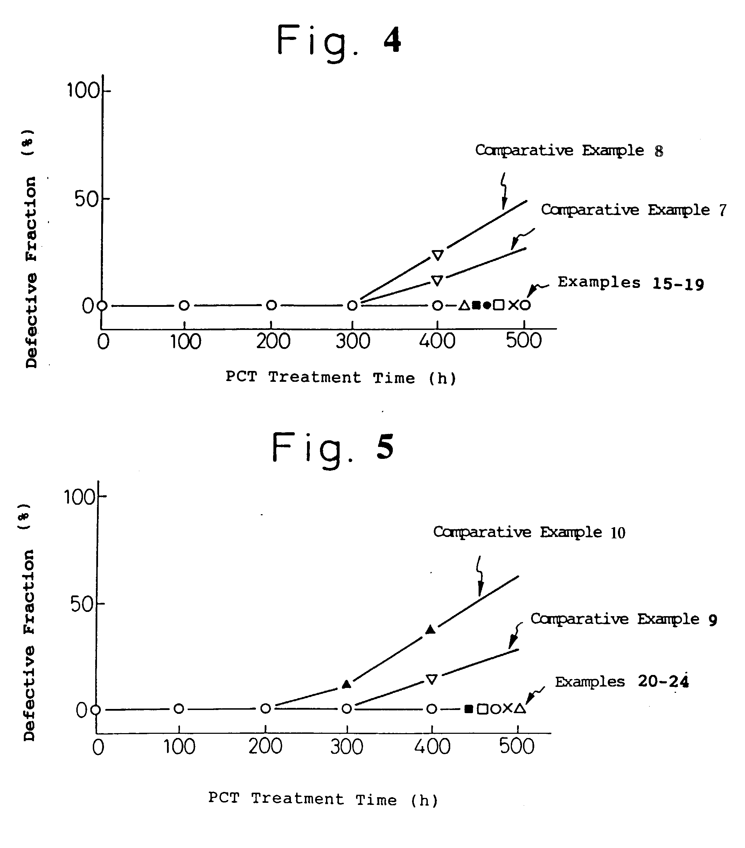

The results are shown in Table 3 and in Fig. 4. It is clearly noted from these results that the addition of the polystyrene/polybutadiene/polyester terminal block copolymer in an amount of from 5 to 80 parts by weight improved the flexibility of a cured resin product, and that the use of the polyallyl phenol as a curing agent allowed the production of a resin composition having an excellent hydrophobicity.

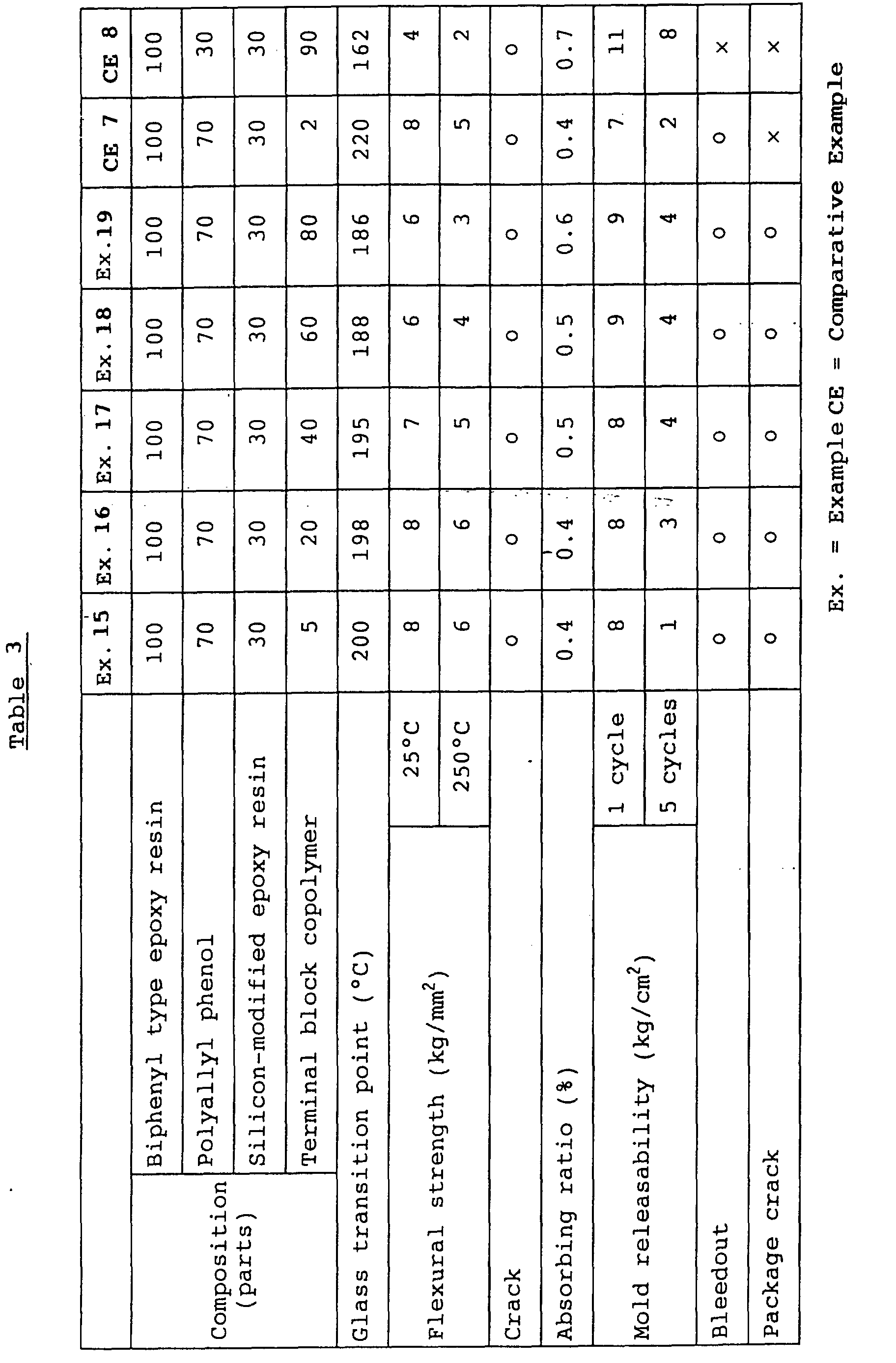

### Examples 20 and 24 and Comparative Examples 9 and 10:

The experiments covered herein represent cases using an epoxy resin composition comprising an epoxy resin, a polyallyl phenol, a silicon-modified epoxy resin, and an ethylene/propylene type terpolymer.

The following raw materials were used in these experiments.
- Epoxy resin:: A biphenyl type epoxy resin (produced by Yuka-Shell Epoxy K.K. and marketed under product code "YX-4000H")
- Polyallyl phenol:: A product of Mitsubishi Petro-Chemical Co., Ltd. marketed under product code "SH-150AR"
- Silicon-modified epoxy resin:: A product of Dai-Nippon Ink & Chemicals, Inc. marketed under product code "SIN-620"
- Ethylene/propylene type terpolymer:: A product of Japan Synthetic Rubber Co., Ltd. marketed under product code "JSR57P"

A pertinent composition was prepared by placing these raw materials in a varying ratio indicated in Table 4 in a pressure kneader and kneading them simultaneously therein. The test pieces of these compositions and tests for various properties were carried out in the same manner as in Examples 15 to 19.

The results are shown in Table 4 and in Fig. 5. It is clearly noted from these results, that the addition of the ethylene/propylene type terpolymer in an amount of from 5 to 80 parts by weight improved the flexibility of a cured resin product, and that the use of the polyallyl phenol as a curing agent allowed the production of a resin composition having an excellent hydrophobicity.

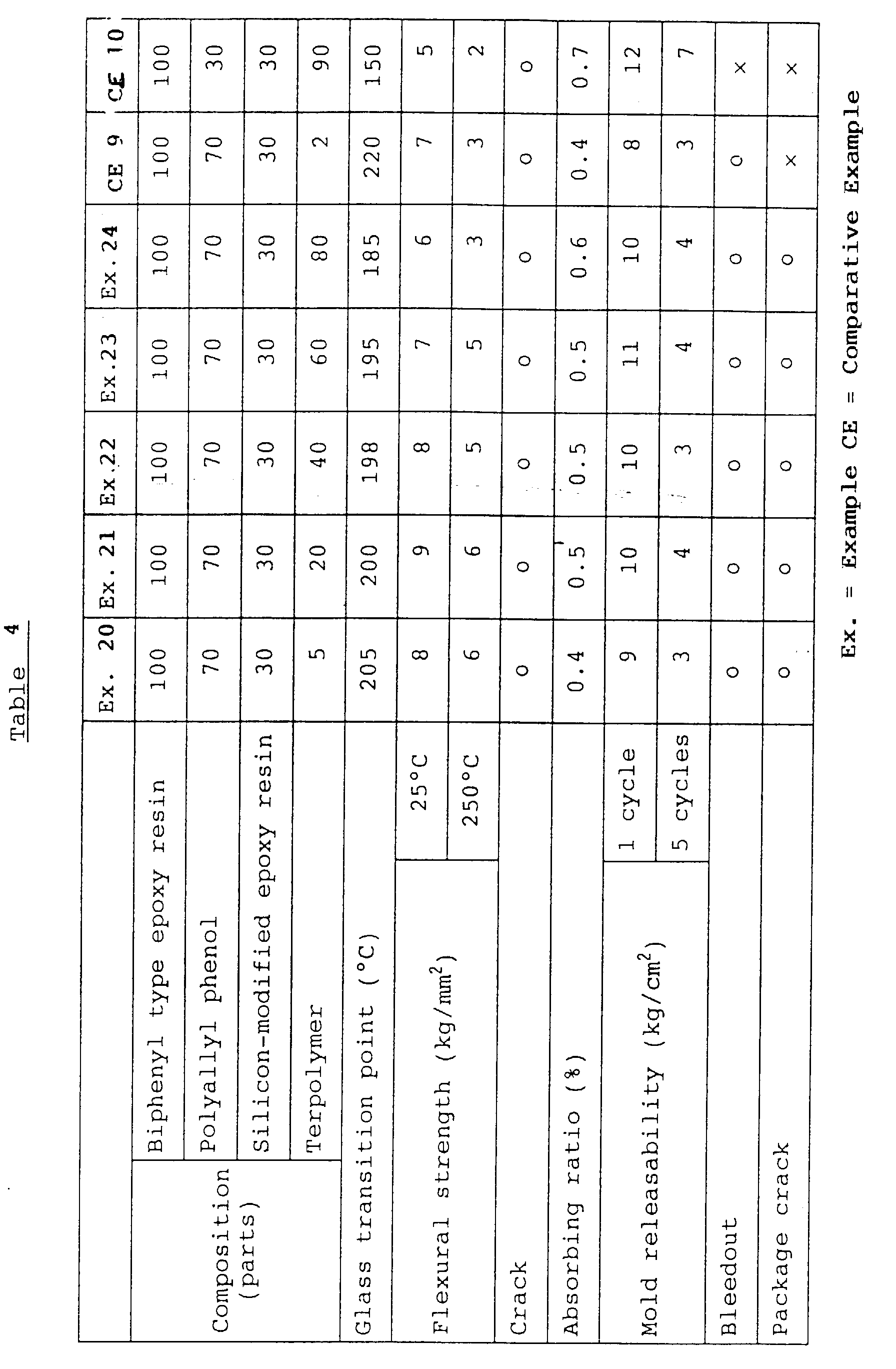

### Examples 25 to 29 and Comparative Examples 11 and 12:

The experiments covered herein represent cases using an epoxy resin composition comprising an epoxy resin, a polyallyl phenol, a silicon-modified epoxy resin, and an ethylene/α-olefin copolymer.

The following raw materials were used in these experiments.
- Epoxy resin:: A biphenyl type epoxy resin (produced by Yuka-Shell Epoxy K.K. and marketed under product code "YX-4000H")
- Polyallyl phenol:: A product of Mitsubishi Petro-Chemical Co., Ltd. marketed under product code "SH-150AR"
- Silicon-modified epoxy resin:: A product of Dai-Nippon Ink & Chemicals, Inc. marketed under product code "SIN-620"
- Ethylene/α-olefin copolymer:: A product of Mitsui Petrochemical Industries, Ltd. marketed under the trademark "Tafmer-P P-0280"

A pertinent composition was prepared by placing these raw materials in a varying ratio indicated in Table 5 in a pressure kneader and kneading them simultaneously therein.

The test pieces of these compositions and the tests for various properties were carried out in the same manner as in Examples 15 to 19.

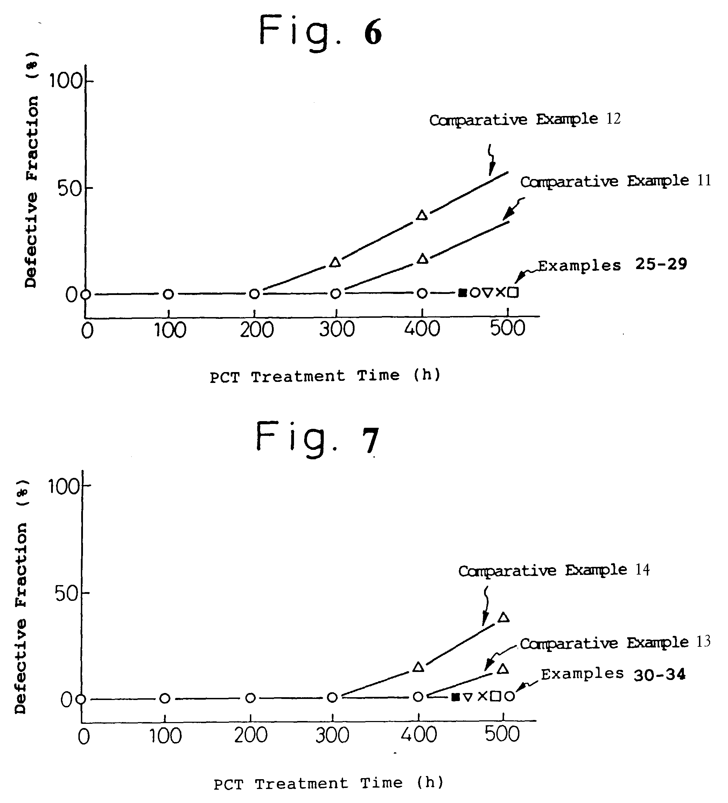

The results are shown in Table 5 and Fig. 6. It is clearly noted from these results, that the addition of the ethylene/α-olefin copolymer in an amount of 5 to 80 parts by weight improved the flexibility of a cured resin product, and that the use of the polyallyl phenol as a curing agent allowed the production of a resin composition excellent having an hydrophobicity.

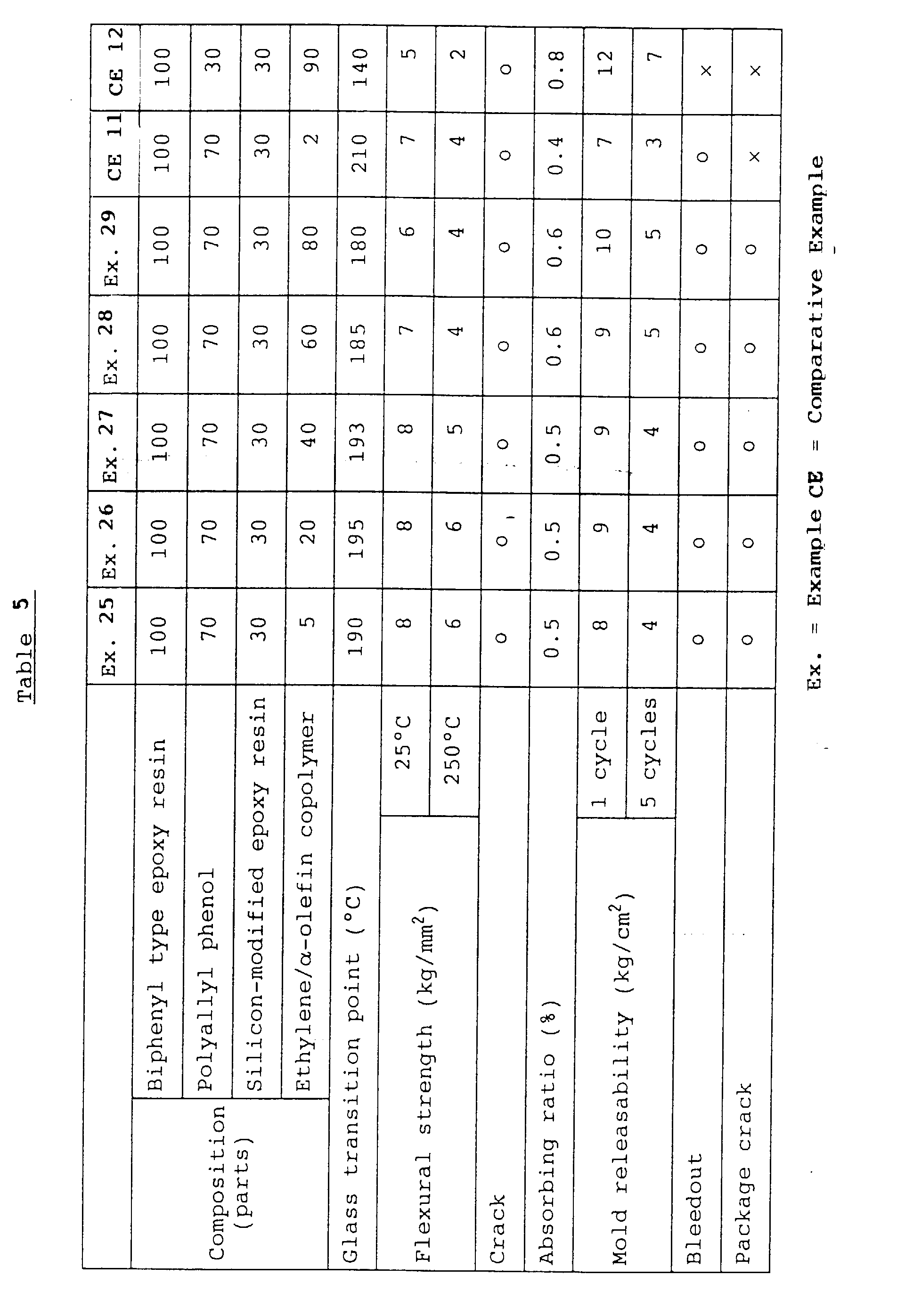

### Examples 30 to 34 and Comparative Examples 13 and 14:

The experiments covered herein represent cases using an epoxy resin composition comprising an epoxy resin, a polyallyl phenol, a silicon-modified epoxy resin, and an epoxy functional group-containing silicone powder.

The following raw materials were used in these experiments:
- Epoxy resin:: A biphenyl type epoxy resin (produced by Yuka-Shell Epoxy K.K. and marketed under product code "YX-4000H")
- Polyallyl phenol:: A product of Mitsubishi Petro-Chemical Co., Ltd. marketed under product code "SH-150AR"
- Silicon-modified epoxy resin:: A product of Dai-Nippon Ink & Chemicals, Inc. marketed under product code "SIN-620"
- Epoxy-functional group containing silicone powder:: A product of Toray-Dow Corning K.K. marketed under the trademark "Torayfil E-601"

A pertinent composition was prepared by placing these raw materials in a varying ratio indicated in Table 6 in a pressure kneader and kneading them simultaneously therein. The test pieces of these compositions and the tests for various properties were carried out in the same manner as in Examples 15 to 19.

The results are shown in Table 6 and in Fig. 7. It is clearly noted from these results that the addition of the epoxy functional group-containing silicone powder in an amount of from 5 to 80 parts by weight improved the flexibility of a cured resin product, and that the use of the polyallyl phenol as a curing agent allowed the production of a resin composition having an excellent hydrophobicity.

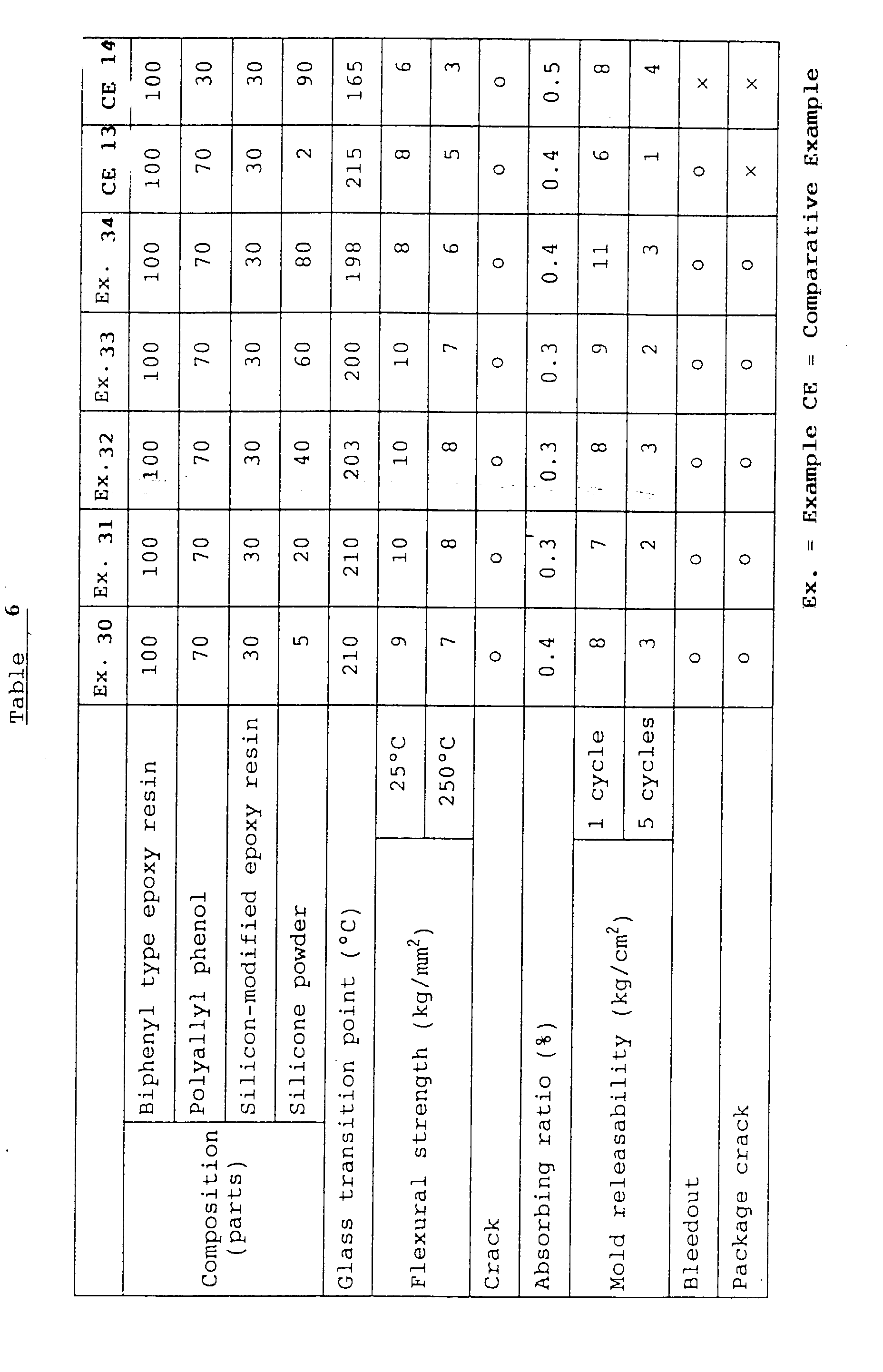

### Examples 35 to 39 and Comparative Examples 15 and 16:

The experiments covered herein represent cases using an epoxy resin composition comprising an epoxy resin, a polyallyl phenol, a silicon-modified epoxy resin, and silica.

The following raw materials were used in these experiments.
- Epoxy resin:: A biphenyl type epoxy resin (produced by Yuka-Shell Epoxy K.K. and marketed under product code "YX-4000H")
- Polyallyl phenol:: A product of Mitsubishi Petro-Chemical Co., Ltd. marketed under product code "SH-150AR"
- Silicon-modified epoxy resin:: A product of Dai-Nippon Ink & Chemicals, Inc. marketed under product code "SIN-620"
- Silica:: A product of Tatsumori K.K. marketed under product code "RD-8"

A pertinent composition was prepared by placing these raw materials in a varying ratio indicated in Table 7 in a pressure kneader and kneading them simultaneously therein. The test pieces of these compositions and tests for various properties were carried out in the same manner as in Examples 15 to 19. The compositions were tested further for viscosity by the flow tester method.

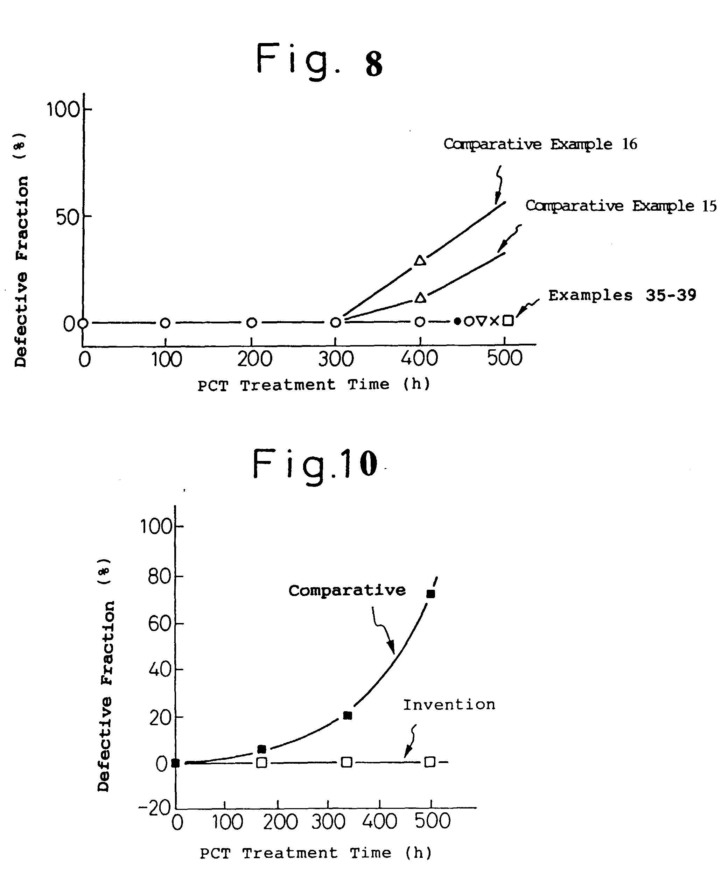

The results are shown in Table 7 and in Fig. 8. It is clearly noted from these results that the addition of silica in an amount of from 30 to 85% by weight improved the mechanical strength of a cured resin product, and that the use of the polyallyl phenol as a curing agent allowed the production of a resin composition having an excellent hydrophobicity.

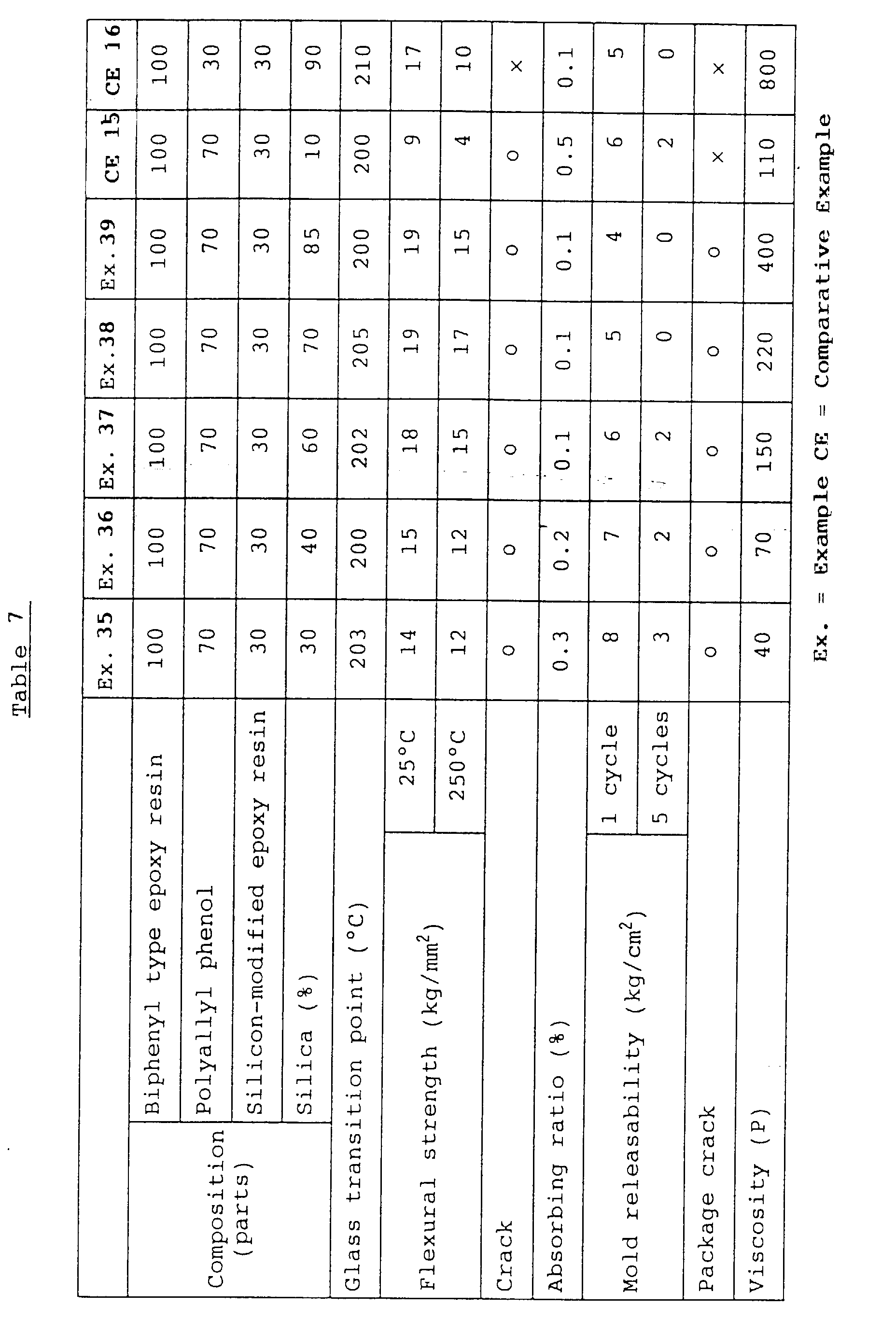

The relationship between the time for the PCT treatment (h) and the fraction which is defective (%) can be easily understood from the accompanying drawings. The products of the comparative examples (cresol novolak type epoxy/phenol novolak type) and the products of this invention (biphenyl type epoxy/polyallyl phenyl type) are compared with respect to this relationship in Fig. 10. It can be understood from the results of the diagram that the products of this invention have an excellent moistureproofing.

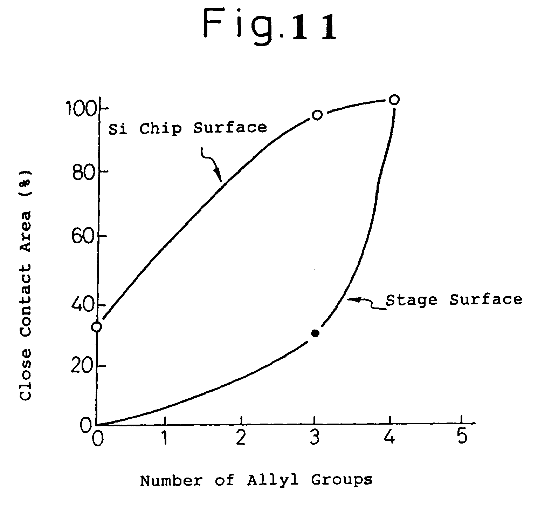

Fig. 11 is a graph showing the effect produced by the number of allyl groups in the polyallyl phenol as a curing agent on the area of close contact. More specifically, this diagram shows the number of allyl groups contained in one benzene ring as a function of the degree of close contact between resin/silicon (Si) chips and resin/stage surface (rear surface) of a 100 pin flat type package after immersion in the solder bath for an absorption of humidity. It is noted from this diagram that the hydrophobicity and freedom from stress were improved and the close contact was enhanced proportionally with the increase of the number of allyl groups.

### INDUSTRIAL APPLICABILITY

This invention can be advantageously utilized for the production of a resin composition having an excellent heatproofing, toughness, flexibility, cracking resistance, mold releasability, and hydrophobicity.

## Claims

1. An epoxy resin composition containing an epoxy resin and a polyallyl phenol curing agent possessing in the repeating unit thereof a plurality of component units represented by the following formula I: 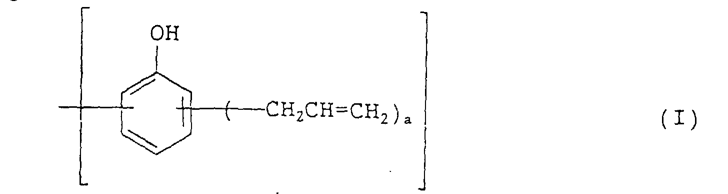 wherein a is an integer of 1 to 4 inclusive,
characterized in that said epoxy resin is a biphenyl epoxy resin, said polyallyl phenol curing agent is contained in an amount of 30 to 120 parts by weight based on 100 parts by weight of said biphenyl epoxy resin, said epoxy resin composition further contains as a flexibility-imparting agent a silicon-modified epoxy resin in an amount of 5 to 80 parts by weight based on 100 parts by weight of said biphenyl epoxy resin and an additional component selected from butadiene/acrylonitrile copolymer, polystyrene /polybutadiene/polystyrene terminal block copolymer, ethylene/propylene type terpolymers, ethylene/α-olefin copolymers, and epoxy group-containing silicone rubber powder is either absent or is present in an amount of from 5 to 80% by weight, based on the weight of the said biphenyl epoxy resin.

2. A composition according to claim 1, wherein said polyallyl phenol is a polyallyl phenol represented by the following formula II: 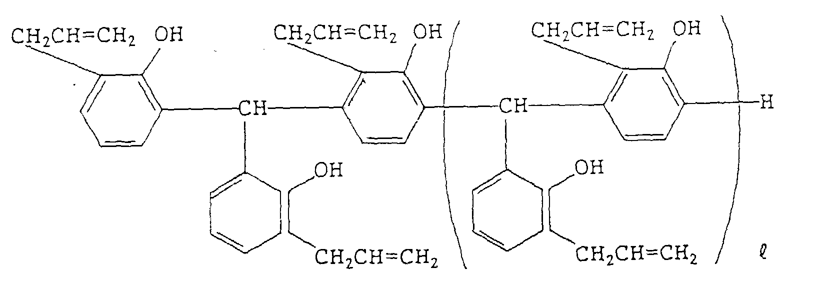 (wherein ℓ is 0 or an integer of 1 to 4 inclusive).

3. A composition according to claim 1, wherein said polyallyl phenol is a polyallyl phenol represented by the following formula III: 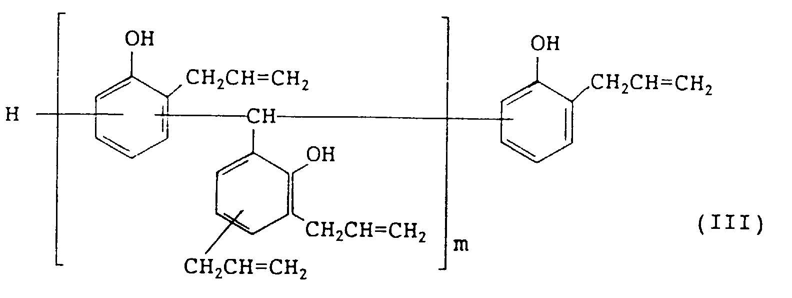 (wherein m is an integer of 1 to 4 inclusive)

4. A composition according to claim 1, wherein said polyallyl phenol is a polyallyl phenol represented by the following formula IV: 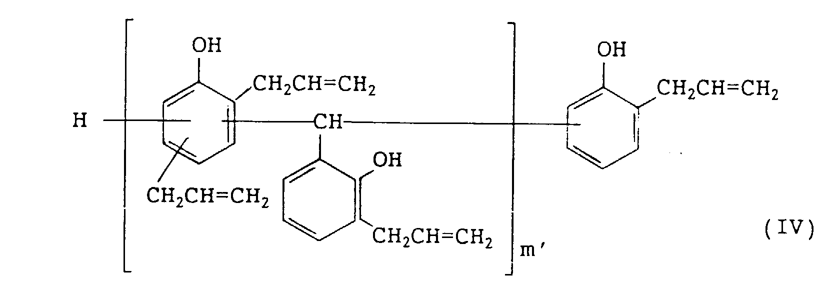 (wherein m' is an integer of 1 to 4 inclusive).

5. A composition according to claim 1, wherein said polyallyl phenol is a polyallyl phenol represented by the following formula V: 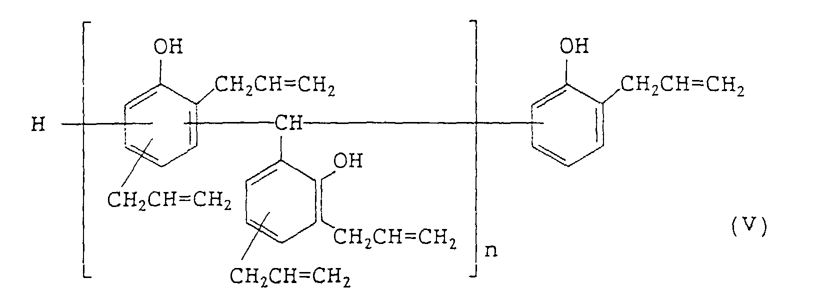 wherein n is an integer of 1 to 4 inclusive.

6. A composition according to any preceding claim, which further contains as a filler a powdery inorganic component in an amount of from 30 to 85% by weight, based on the total amount of the composition.

## Patentansprüche

1. Epoxyharz-Zusammensetzung, welche ein Epoxyharz und ein Polyallylphenol-Aushärtungsmittel enthält, das in der Repetiereinheit davon eine Vielzahl von Komponenteneinheiten der folgenden Formel (I) aufweist: 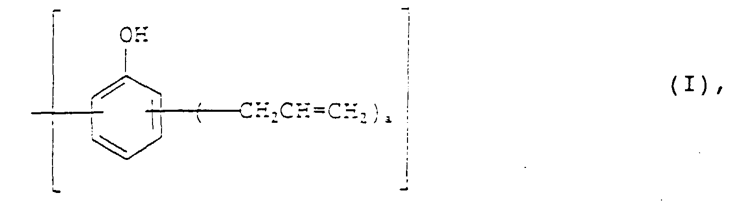 worin a eine ganze Zahl von 1 bis einschließlich 4 ist, dadurch gekennzeichnet, daß das Epoxyharz ein Biphenylepoxyharz ist, das Polyallylphenol-Aushärtungsmittel in einer Menge von 30 bis 120 Masseteilen pro 100 Masseteile des Biphenylepoxyharzes enthalten ist, die Epoxyharz-Zusammensetzung ferner als Flexibilität verleihendes Mittel ein Silicium-modifiziertes Epoxyharz in einer Menge von 5 bis 80 Masseteilen pro 100 Masseteile des Biphenylepoxyharzes und eine zusätzliche Komponente enthält, die aus Butadien/Acrylnitril-Copolymer, Polystyrol/Polybutadien/Polystyrol-terminalem Blockcopolymer, Terpolymeren vom Ethylen/Propylen-Typ, Ethylen/α-Olefin-Copolymeren ausgewählt ist, und eine Epoxy-Gruppe enthaltendes Silikonkautschuk-Pulver entweder fehlt oder in einer Menge von 5 bis 80 Masse-%, bezogen auf die Masse des Biphenylepoxyharzes, vorliegt.

2. Zusammensetzung nach Anspruch 1, worin das Polyallylphenol ein Polyallylphenol der folgenden Formel (II) ist: 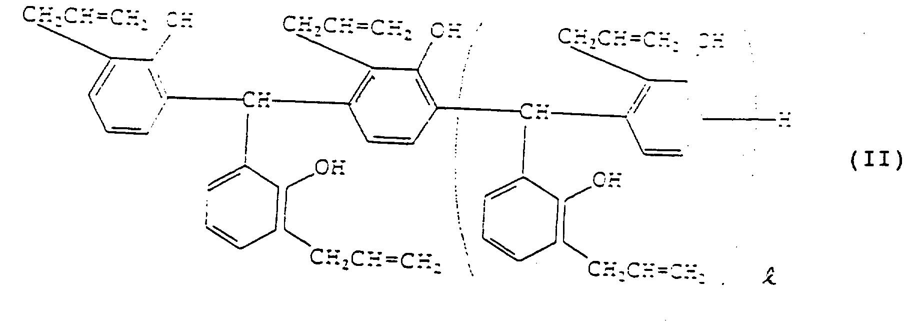 (worin ℓ Null oder eine ganze Zahl von 1 bis einschließlich 4 ist).

3. Zusammensetzung nach Anspruch 1, worin das Polyallylphenol ein Polyallylphenol der folqenden Formel (III) ist: 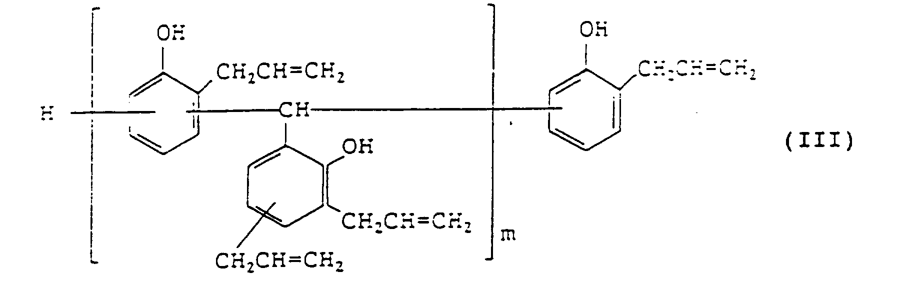 (worin m eine ganze Zahl von 1 bis einschließlich 4 ist).

4. Zusammensetzung nach Anspruch 1, worin das Polyallylphenol ein Polyallylphenol der folgenden Formel (IV) ist: 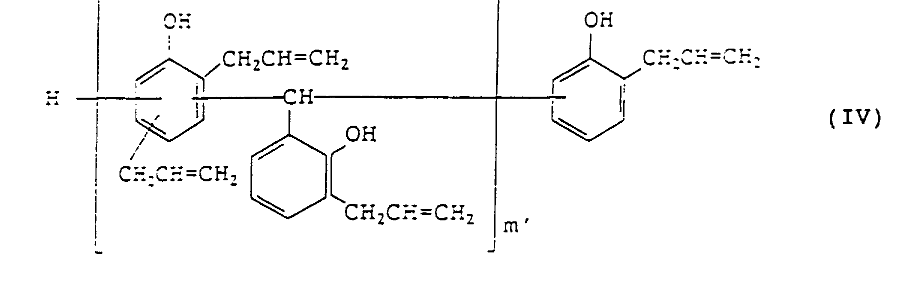 (worin m' eine ganze Zahl von 1 bis einschließlich 4 ist).

5. Zusammensetzung nach Anspruch 1, worin das Polyallylphenol ein Polyallylphenol der folgenden Formel (V) ist: 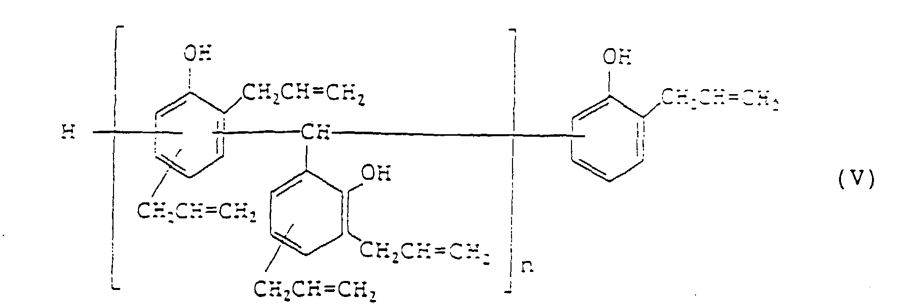 (worin n eine ganze Zahl von 1 bis einschließlich 4 ist).

6. Zusammensetzung nach einem der vorhergehenden Ansprüche, welche ferner als Füllstoff eine pulverförmige anorganische Komponente in einer Menge von 30 bis 85 Masse-%, bezogen auf die Gesamtmasse der Zusammensetzung, enthält.

## Revendications

1. Composition de résine époxy comprenant une résine époxy et un agent de réticulation de polyallylphénol possédant dans l'unité répétitive de celle-ci de nombreuses unités de constituants représentées par la formule I suivante : 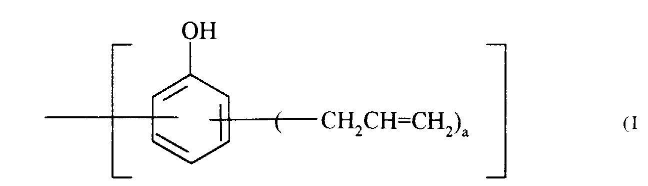 dans laquelle a est un nombre entier de 1 à 4 inclus, caractérisée en ce que ladite résine époxy est une résine époxy de biphényle, ledit agent de réticulation de polyallylphénol est contenu dans une quantité de 30 à 120 parties en poids rapportées à 100 parties en poids de ladite résine époxy de biphényle, ladite composition de résine époxy contient en outre comme agent communiquant la flexibilité une résine époxy modifiée au silicium dans une quantité de 5 à 80 parties en poids rapportées à 100 parties en poids de ladite résine époxy de biphényle et un constituant supplémentaire choisi parmi un copolymère de butadiène/acrylonitrile, un copolymère séquencé terminal de polystyrène/polybutadiène/polystyrène, des terpolymères de type éthylène/propylène, des copolymères d'éthylène/oléfine-α et de la poudre de caoutchouc de silicone contenant un groupe époxy est soit absente, soit présente dans une quantité de 5 à 80 % en poids, rapportés au poids de ladite résine époxy de biphényle.

2. Composition selon la revendication 1, dans laquelle ledit polyallylphénol est un polyallylphénol représenté par la formule II suivante : 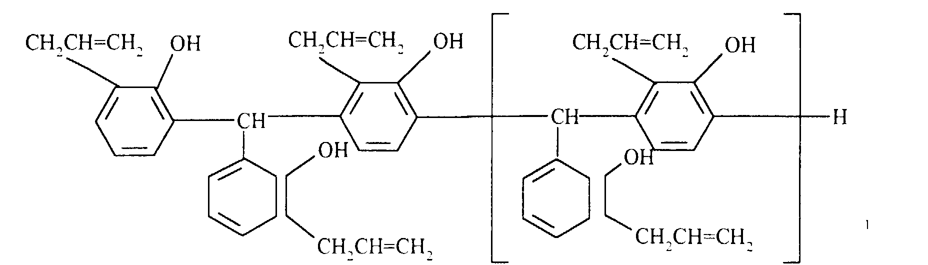 (dans laquelle 1 est égal à 0 ou à un nombre entier compris entre 1 et 4 inclus).

3. Composition selon la revendication 1, dans laquelle ledit polyallphénol est un polyallylphénol représenté par la formule III suivante : 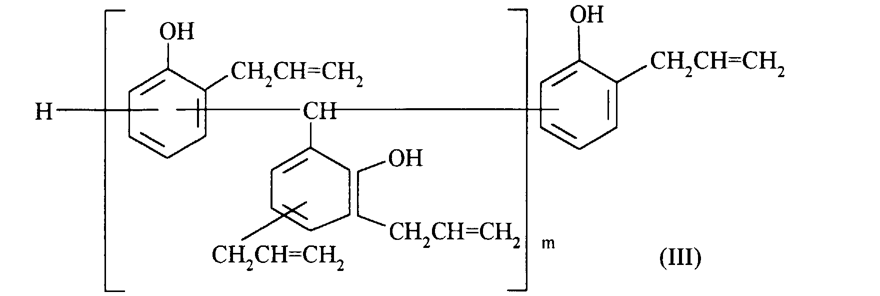 dans laquelle m est un nombre entier compris entre 1 et 4 inclus).

4. Composition selon la revendication 1, dans laquelle ledit polyallylphénol est un polyallylphénol représenté par la formule IV suivante : 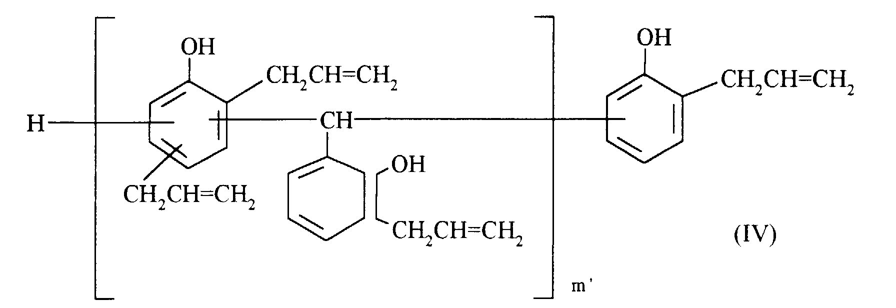 (dans laquelle m' est un nombre entier compris entre 1 et 4 inclus).

5. Composition selon la revendication 1, dans laquelle ledit polyallylphénol est un polyallylphénol représenté par la formule V suivante : 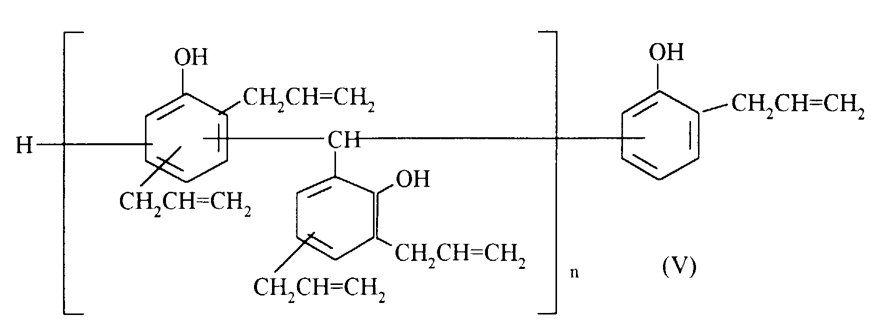 dans laquelle n est un nombre entier compris entre 1 et 4 inclus.

6. Composition selon l'une quelconque des revendications précédentes qui contient en outre comme charge un constituant inorganique sous forme de poudre dans une quantité de 30 à 85 % en poids, rapportés à la quantité totale de la composition.
